(19) Europäisches Patentamt
European Patent Office
Office européen des brevets

(11) EP 0 537 909 B1

(12) **EUROPEAN PATENT SPECIFICATION**

(45) Date of publication and mention of the grant of the patent:
**22.01.1997 Bulletin 1997/04**

(51) Int Cl.[6]: **C07C 69/757**, C07C 67/34, C07C 67/343, A01N 37/42, C07C 49/697, C07C 45/65

(21) Application number: **92308507.0**

(22) Date of filing: **18.09.1992**

(54) **3-(Unsubstituted or substituted benzyl)-1-alkyl-2-oxocyclopentane carboxylic acid alkyl ester derivatives, and their preparation and use**

3-(unsubstituiertes oder substituiertes Benzyl)-1-Alkyl-2-oxocyclopentancarbonsäurealkylesterderivate, und ihre Herstellung und ihre Anwendung

Dérivés alkyl esters de l'acide (benzyl, substitué ou non)-3 -alkyl-1-oxo-2-cyclopentane carboxylique, leur préparation et leur utilisation

(84) Designated Contracting States:
**BE DE GB NL**

(30) Priority: **18.09.1991 JP 267073/91**

(43) Date of publication of application:
**21.04.1993 Bulletin 1993/16**

(73) Proprietor: **KUREHA CHEMICAL INDUSTRY CO., LTD.**
**Chuo-ku Tokyo 103 (JP)**

(72) Inventors:
- **Ichinose, Isao**
  **Iwaki-shi, Fukushima (JP)**
- **Minoguchi, Masanori**
  **Kiyose-shi, Tokyo (JP)**
- **Kumazawa, Satoru**
  **Iwaki-shi, Fukushima (JP)**
- **Yoshida, Eyji**
  **Iwaki-shi, Fukushima (JP)**

(74) Representative: **Geering, Keith Edwin**
**REDDIE & GROSE**
**16 Theobalds Road**
**London WC1X 8PL (GB)**

(56) References cited:
**EP-A- 0 267 778** **EP-A- 0 413 448**

- **CHEMICAL ABSTRACTS, vol. 113, no. 9, 1990, Columbus, Ohio, US; abstract no. 78396, page 785, column 2; & JP-A-2 072 176**

EP 0 537 909 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

## Description

The present invention relates to 3-(unsubstituted or substituted benzyl)-1-alkyl-2-oxocyclopentane carboxylic acid alkyl ester derivatives, preparation of the same, fungicide containing the same, and use thereof as intermediate compounds.

EP-A-294,222 US-A-4,938,792 (EP-A-267,778), and EP-A-341,954 disclose 2-(unsubstituted or substituted benzyl)-5-alkylcyclopentanone derivatives (VII')

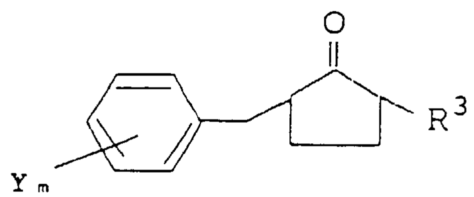

(VII')

(where

$R^3$ is a lower alkyl group;
Y is a halogen atom, a cyano group, an alkyl group, a haloalkyl group or a nitro group; and
m is 0 or an integer of 1 to 5 and when m is greater

than 1 each Y can be the same or different), which can be employed as intermediate compounds for pesticides, medicines, and the like.

US-A-4,938,792 contains a detailed description of reaction schemes (i) and (ii) below for the preparation of a 2-(unsubstituted or substituted benzyl)-5-alkylcyclopentanone derivative (VII")

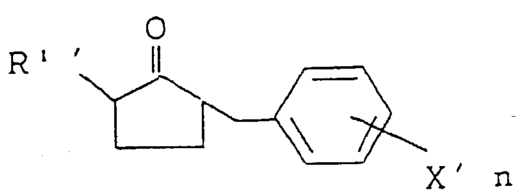

(VII")

(wherein

$R^{1'}$ is a $C_1$ - $C_5$ alkyl group;
X' is a halogen atom, a $C_1$ - $C_5$ alkyl group or a phenyl group; and
n is 0, 1 or 2).

The reaction scheme (i) involves benzylating a 2-oxocyclopentane carboxylic acid alkyl ester derivate (II') with an unsubstituted or substituted benzyl halide (III'); alkylating the resulting benzylated product (IV') with an alkyl halide; hydrolyzing the ester group of the resulting alkylated product (VIII); and decarboxylating the hydrolyzate to give product (VII").

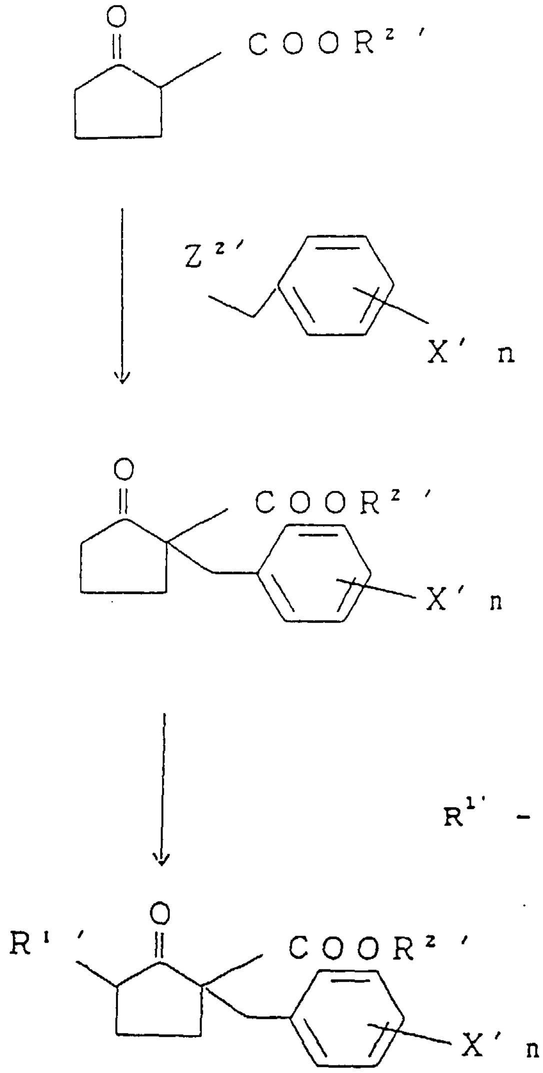

(II')

(III')

(IV')

$R^{1'} - Z^{1'}$ (VI')

(VIII).

(where

| | |
|---|---|
| $R^{2'}$ | is a $C_1$ - $C_3$ alkyl group; |
| $Z^{1'}$ and $Z^{2'}$ | are each a halogen atom; and |
| $R^{1'}$, X' and n | have the same meanings as above). |

The reaction scheme (ii) for the preparation of compound (VII") involves benzylating 3-alkyl-2-oxocyclopentane carboxylic acid alkyl ester derivative (IX) with unsubstituted or substituted benzyl halide (III'); hydrolyzing the ester group of resulting benzylated compound (VIII); and decarboxylating the hydrolyzate to give the compound (VII")

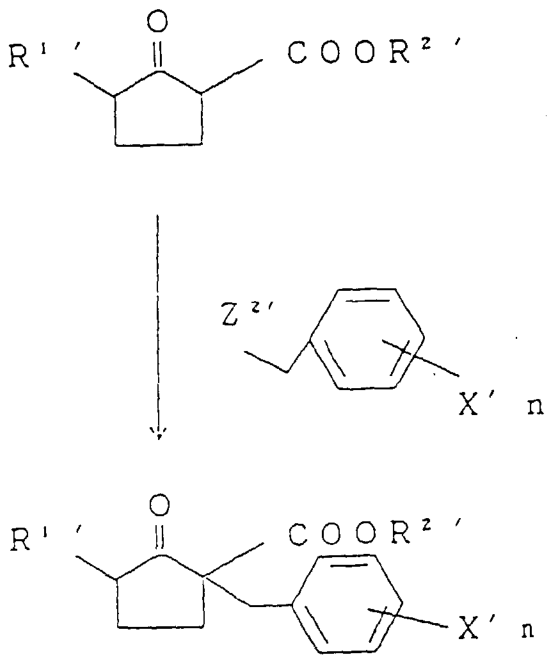

(IX)

(III')

(VIII)

(where $R^{1'}$, $R^{2'}$, X', $Z^{2'}$ and n have the same meanings as above).

In order to provide good in yield these schemes, the alkylation in scheme (i) should be carried out using a strong base such as sodium hydride or the like in an aprotic solvent; and that in scheme (ii) should be carried out by alkylating a 2-oxocyclopentane carboxylic acid alkyl ester derivative (II′) with alkyl halide (VI′) in the presence of a base, subjecting the resulting compound (X) to rearrangement in a lower alcohol in the presence of an alkali metal lower alkoxide; and isolating the resulting compound (IX):

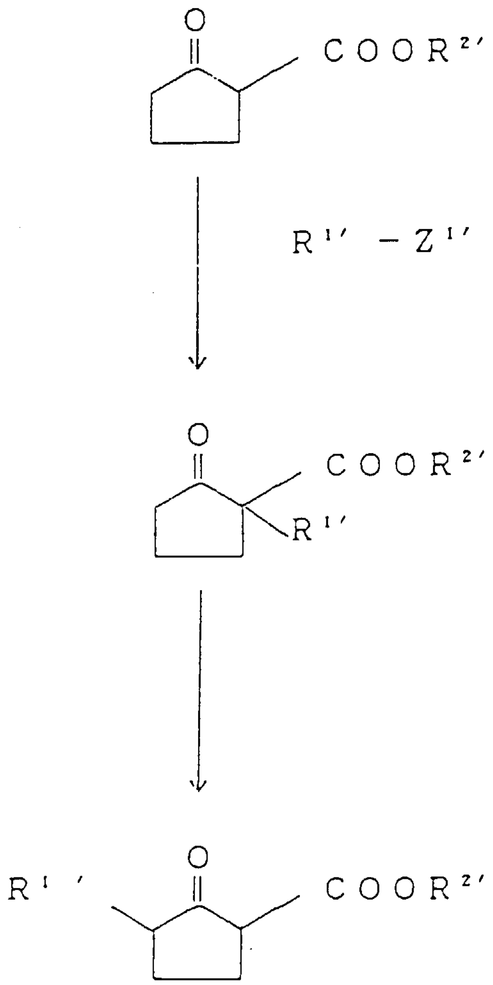


(II')

(VI')

(X)

rearrangement

(IX)

(where $R^{1\prime}$, $R^{2\prime}$, $X'$, $Z^{2\prime}$ and n have the same meanings as above).

In order to provide precursors of the compounds (VII′) and (VIII) in high yield, a strong base such as sodium hydride or the like should be employed or the intermediate compounds should be isolated and purified from the reaction mixtures.

Particular care is required, however, in using sodium hydride in large quantity because hydrogen is evolved during the reaction. Further, the isolation and purification of the intermediate compounds require additional steps in the reaction scheme.

Hence, strong demands have been made to provide a method in which the yield of the product is not reduced, a base easily and readily handled can be employed, and isolation and purification of intermediate compounds are not required so that the number of reaction steps is reduced.

The present invention provides a precursor of 2-(unsubstituted or substituted benzyl)-5-alkylcyclopentanone derivative (VII) below which can be prepared by a method in which an easily handled base can be employed, intermediate isolation and purification is not required, and the number of reaction steps is small.

The invention provides methods for the preparation of the precursor from compounds (IV) and (II) below.

In addition, the present invention provides a method capable of easily producing derivative (VII) from the precursor.

The present invention also provides novel use of the precursor as a fungicide, particularly an agricultural and horticultural fungicide, in addition to use as an intermediate compound for pesticides, medicines and the like.

We have made extensive research and review to find a base more easily handled than sodium hydride and to reduce the reaction steps without necessitating the isolation and purification of any intermediate compound in preparing the precursor of derivative (VII).

As a result, it has been found that 3-(unsubstituted or substituted benzyl)-1-alkyl-2-oxocyclopentane carboxylic acid alkyl ester derivative (I) can be utilized as a precursor for compound (VII) in the following reaction scheme (B):

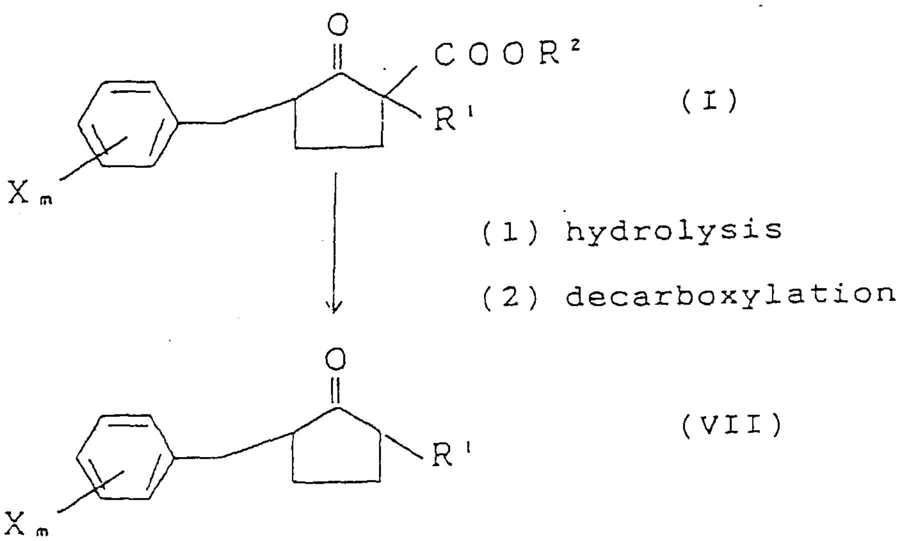


(I)

(VII)

where $R^1$ and $R^2$ are the same or different $C_1$ - $C_5$ alkyl groups, m is 0 or an integer from 1 to 5, and the or each X, when present, is selected independently from halogen, cyano, $C_1$ - $C_5$ alkyl, $C_1$-$C_5$ haloalkyl, phenyl and nitro groups, these symbols hereinafter having the same meanings throughout unless otherwise specified.

Further, compound (I) can be prepared in high yield by subjecting derivative (II) to benzylation with alkali metal lower alkoxide (III), rearrangement, and alkylation, without isolation and purification of any intermediate compound, in the following reaction scheme (A):

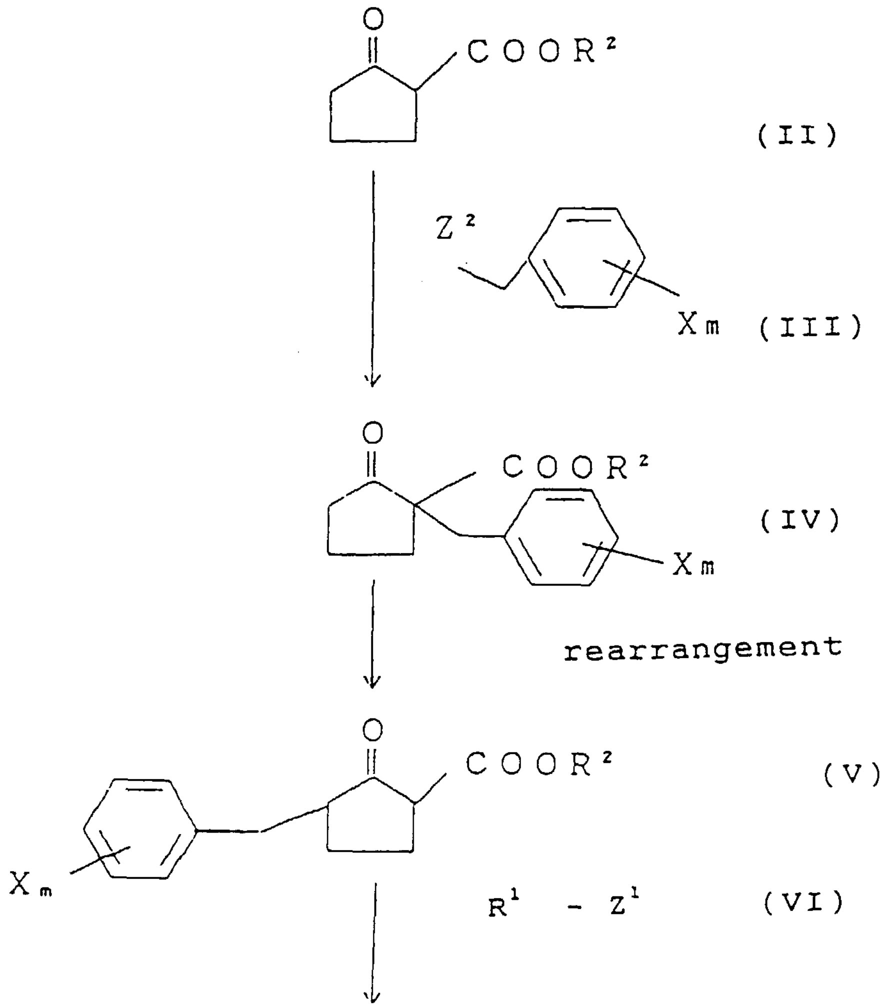

(II)
(III)
(IV)
(V)
(VI)

(I)

where $Z^1$ and $Z^2$ are each a halogen atom.

The present invention provides the novel derivatives (I)

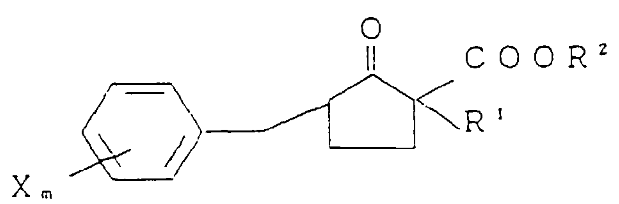
(I)

and a method for preparation thereof by continuous rearrangement and alkylation as above, wherein the rearrangement is carried out to convert derivative (IV) into derivative (V) in the presence of alkali metal lower alkoxide in lower alcohol; and wherein the alkylation is carried out to alkylate resulting compound (V) with the alkyl halide (VI), after distillation of the lower alcohol.

Further, the present invention provides a method for continuous benzylation, and said rearrangement and alkylation as indicated above, wherein the benzylation is carried out to benzylate derivative (II) with unsubstituted or substituted benzyl halide (III) in the presence of alkali metal base.

In addition, the present invention provides a method for the preparation of derivative (VII) by hydrolyzing derivative (I) and then decarboxylating the hydrolyzate.

Furthermore, the present invention provides a fungicide containing derivative (I) as an active ingredient.

The substituents $R^1$ and $R^2$ may be identical to or different from each other and each may be a $C_1$ - $C_5$ monovalent, straight-chained or branched saturated hydrocarbon residue; they are preferably $C_1$ - $C_4$ alkyl groups and more preferably selected from methyl, ethyl, propyl, and isopropyl. The term "halogen" herein means, for example, chlorine, bromine, fluorine and the like. Preferred is chlorine, most preferably joined at the 4-position of the benzene ring. "Lower" herein means $C_1$ - $C_5$ where the context allows; "alkyl" unless otherwise specified indicates lower alkyl, e.g. as preferred for $R^1$ and $R^2$; "haloalkyl" means such lower alkyl with at least one hydrogen atom substituted with halogen, preferably fluorine. Symbol "m" is preferably 0 or 1.

The deriveratives (I) according to the invention include the specific compounds in Table 1.

TABLE 1

| Compound Nos. | Substituents of General Formula (I) | | |
|---|---|---|---|
| | $R^1$ | $R^2$ | Xm |
| I-1 | $CH_3$ | $CH_3$ | 4-Cl |
| I-2 | $(CH_3)_2CH$ | $CH_3$ | 4-Cl |

Note:
The term "4-Cl" means substitution of chlorine in the 4-position of the phenyl group.

In one preparation of derivative (I) from derivative (II) by reaction scheme (A) above, an alkali metal base is added preferably at the rate of 1.0 to 1.2 moles equivalent to the derivative (II). When alkali metal lower alkoxide is employed, lower alcohol is azeotropically distilled off with an appropriate solvent such as toluene and the compound (II) is then benzylated with the unsubstituted or substituted benzyl halide (III). If the halide is not an iodide, it is preferred to use an alkali metal iodide in a catalytic amount. The alkali metal alkoxide may be selected, for example, from sodium and potassium methoxides, sodium ethoxide, sodium isopropoxide, potassium t-butoxide, and the like.

The solvent to be employed in this reaction may be, for example, an aromatic hydrocarbon such as toluene, xylene, etc., a nitrile such as acetonitrile, etc., an amide such as dimethylformamide, dimethyl acetamide, N-methylpyrrolidone, etc., and the like. The solvents may be employed singly or in combination of two or more.

The reaction may be carried out at temperatures of from approximately 50° C to 100° C, preferably from approximately 70° C to 90° C.

To the reaction mixture containing derivative (IV) obtained by the benzylation are added lower alcohol and alkali metal lower alkoxide at the rate of 1.0 to 1.2 moles equivalent, and the mixture is heated under reflux to carry out the rearrangement into the derivative (V). After the lower alcohol has been distilled off, the derivative (V) is then alkylated with the alkyl halide (VI) to give derivative (I). As the alkyl halide (VI), there may be mentioned, for example, methyl bromide, methyl iodide, ethyl iodide, propyl bromide, isopropyl iodide, n-butyl chloride, isobutyl bromide and the like.

If the halide employed in this alkylation is not an iodide, it is preferred to use a catalytic amount of alkali metal iodide. As the solvent to be employed for the rearrangement, the lower alcohol may be employed singly or in combination with other organic solvents as described hereinabove, such as an aromatic hydrocarbon or the like.

After completion of the rearrangement, an aromatic hydrocarbon such as toluene is added, and the lower alcohol is distilled off; even if the aromatic hydrocarbon is left in the reaction mixture, it does not adversely affect the alkylation which follows.

The alkylation may be carried out in the solvent, and the solvent to be employed therefor may include, for example, a nitrile solvent such as acetonitrile, etc., an amide solvent such as methylformamide, dimethylformamide, dimethyl acetamide, N-methylpyrrolidone, etc., a sulfur-containing compound such as dimethylsulfoxide, sulfolane etc., an alcohol such as t-butyl alcohol, and the like. These solvents may be employed singly or in combination of two or more. The reaction may be carried out at temperatures of from approximately 50° C to 100° C, preferably from approximately 70° C to 90° C.

The resulting derivative (I) may be isolated from the resulting reaction mixture by usual procedures such as column chromatography, recrystallization, and the like. Any such procedure may be employed alone or in combination with other procedures.

The resulting derivative (I) can by hydrolyzed and then decarboxylated to give derivative (VII) with ease and high yield by reaction scheme (B) above.

The hydrolysis and the decarboxylation can be carried out in either acidic or basic conditions.

When the reaction is to be carried out in acidic conditions, it is desired to use acetic acid as a solvent, in addition to water. A catalyst may be employed, and the catalyst may be, for example, an inorganic acid such as hydrochloric acid, hydrobromic acid and the like, The reaction may be carried out at temperatures from approximately 50° C to the reflux point, preferably from approximately 80° C to the reflux point.

When derivative (I) is used as a fungicide it is generally in the form of dust, wettable powder, granules, emulsion

and the like with carriers or other adjuvants. In such cases, the preparations can for example contain one or more of the compounds (I) of the present invention in a total amount of 0.1 % to 95% by weight, preferably 0.5% to 90% by weight, and more preferably 2% to 70% by weight.

The auxiliary agents to be employed for the preparation may include, for example, carrier, diluent, surfactant and the like, which have conventionally been employed as auxiliary agents for the preparation of fungicides. The carrier in a solid form may comprise, for example, talc, kaolin, bentonite, diatomaceous earth, white carbon, clay, and the like; the diluent in a liquid form may comprise, for example, water, xylene, toluene, chlorobenzene, cyclohexane, cyclohexanone, dimethylsulfoxide, dimethylformamide, and alcohol, and the like.

The surfactant is preferably chosen depending upon the forms of the preparations and their effects; an emulsifiable agent may comprise, for example, polyoxyethylene alkyl aryl ether, polyoxyethylene sorbitan monolaurate, and the like; a dispersing agent may comprise, for example, lignin sulfonate, dibutylnaphthalene sulfonate, and the like; and a wetting agent may comprise, for example, an alkyl sulfonate, an alkylbenzene sulfonate, and the like.

The above preparations are classified into those which can be used directly, and those which are used after diluting so as to have a suitable concentration with a diluent such as water, etc. The concentration of the present compounds after diluting is preferred to be 0.001% - 1.0%.

Further, the application dosage of the compound of this invention is e.g, 20 g - 5,000 g, preferably 50 g - 1,000 g, per 1 ha of agricultural or horticultural land such as farm, paddy field, fruit garden, hothouse, etc.

It is of course possible to increase and decrease the concentration and the application dosage beyond the above-mentioned ranges, because they depend upon the form of preparations, method of application, place to be used, target crops. etc.

Compounds (I) according to the present invention may be employed in combination with other active compounds, such as fungicides, bactericides, insecticides, miticides, herbicides, and the like.

A specific description will now be made of examples of the preparation of derivatives (I) according to the present invention, examples of uses as intermediate compounds, examples of preparations and examples of tests.

The present invention is not limited to the following examples.

Preparation Example 1:

1-methyl-2-oxocyclopentane carboxylate (I-1)

To a solution of 2.6671 grams (0.01 mole) of methyl 1-(4-chlorobenzyl)-2-oxocyclopentane carboxylate in 8 ml of anhydrous methanol was added 2.1 ml of sodium methoxide (a 28% by weight methanol solution), and the mixture was heated under reflux for 30 minutes.

To the resulting reaction mixture was added 20 ml of toluene, methanol was azeotropically distilled off under reduced pressure, and 4 ml of anhydrous dimethylformamide (DMF) and 1.5642 grams (0.011 mole) of methyl iodide were added to the resulting mixture.

After raising the temperature to 60° C and stirring for 1 hour, the reaction mixture was pored into water with ice cubes and then extracted with ethyl acetate. The resulting organic layer was washed with 1N-hydrochloric acid and saturated sodium hydrogen carbonate aqueous solution.

After drying over anhydrous sodium sulfate, the solvent was distilled off under reduced pressure, leaving 2.9826 grams of yellow oily material.

The oily material was purified by silica gel column chromatography, yielding methyl 3-(4-chlorobenzyl)-1-methyl-2-oxocyclopentane carboxylate (2.2616 grams, 0.0081 mole).

The percentage yield and physical properties of this compound (I-1) are as follows:

Yield: 81%

Colorless, transparent oily substance

IR (firm, $\nu_{max}$): 2956, 2880, 1756, 1732, 1496, 1452, 1274, 1160, 1094, 1016, 848, 804 $cm^{-1}$

$^1$H-NMR ($CDCl_3$): δ 1.17, 1.35 (s,3H), 1.56-1.85 (m, 2H), 1.90 - 2.12 (m, 1H), 2.28 - 2.71 (m, 3H), 3.06 - 3.15 (m, 1H), 7.08, 7.10 (d, 2H, J=8.30Hz), 7.25 (d, 2H, J=8.30Hz)

Preparation Example 2:

Preparation of methyl 3-(4-chlorobenzyl)-1-isopropyl-2-oxocyclopentane carboxylate (I-2)

To a solution of 2.6671 grams (0.01 mole) of methyl 1-(4-chlorobenzyl)-2-oxocyclopentane carboxylate in 8 ml of anhydrous methanol was added 2.1 ml of sodium methoxide (a 28% by weight methanol solution), and the mixture was heated under reflux for 30 minutes.

To the resulting reaction mixture was added 20 ml of toluene, methanol was azeotropically distilled off under reduced pressure, and 2 ml of anhydrous dimethylformamide (DMF), 0.5 ml of t-butanol, and 2 ml (0.02 mole) of isopropyl iodide were added to the resulting mixture.

After raising the temperature to 60° C and stirring for 5 hours, the reaction mixture was poured into ice water and then extracted with ethyl acetate. The resulting organic layer was washed with 1N-hydrochloric acid and saturated sodium hydrogen carbonate aqueous solution.

After drying over anhydrous sodium sulfate, the solvent was distilled off under reduced pressure, leaving 2.7762 grams of yellow oily material.

The oily material was purified by silica gel column chromatography, yielding methyl 3-(4-chlorobenzyl)-1-isopropyl-2-oxocyclopentane carboxylate (2.4058 grams, 0.0078 mole).

The percentage yield and physical properties of this compound (I-2) are as follows:

Yield: 78%

Colorless, transparent oily substance

IR (firm, $\nu_{max}$): 2960, 2870, 1745, 1720, 1490, 1460, 1250, 1225, 1160, 1095, 1016, 845, 804, 790, 760 $cm^{-1}$

$^1$H-NMR ($CDCl_3$): $\delta$ 0.78, 0.81 (d, 3H, J=7.32Hz), 0.84, 0.87 (d, 3H, J=7.32Hz), 1.5 - 1.8 (m, 2H), 2.01 (m, 1H), 2.2 - 2.4 (m, 2H), 2.5 - 2.7 (m, 2H), 3.08 (dd, 1H, J=13.67, 3.91Hz), 3.62, 3.70 (s, 3H), 7.06, 7.10 (d, 2H, J=8.30Hz), 7.23, 7.26 (d, 2H, J=8.30Hz)

Preparation Example 3:

Preparation of methyl 3-(4-chlorobenzyl)-1-isopropyl-2-oxocyclopentane carboxylate (I-2)

To a solution of 3.41 grams (0.024 mole) of methyl 2-oxocyclopentane carboxylate in 20 ml of anhydrous methanol was added 5.3 ml of sodium methoxide (a 28% by weight methanol solution), and the mixture was heated under reflux for 30 minutes.

To the resulting reaction mixture was added 68 ml of toluene, methanol was azeotropically distilled off under reduced pressure, and 10 ml of toluene, 2 ml of anhydrous dimethylformamide (DMF), 3.9495 grams (0.025 mole) of p-chlorobenzyl chloride, and 0,3965 mg of potassium iodide were added to the resulting mixture.

After the solvent was distilled off, 10 ml of anhydrous methanol and 5.3 ml of sodium methoxide (a 28% by weight methanol solution) were added, followed by heating the resulting mixture under reflux for 30 minutes.

To the resulting reaction mixture was added 70 ml of toluene, and thereafter methanol was azeotropically distilled off, followed by the addition of 17.5 ml of anhydrous dimethylformamide (DMF), 2 ml of t-butanol and 4.8 ml (0.048 mole) of isopropyl iodide.

After raising the temperature to 60° C and stirring for 5 hours, the reaction mixture was poured into water with ice cubes and extracted with ethyl acetate. The resulting organic layer was washed with 1N-hydrochloric acid and saturated sodium hydrogen carbonate aqueous solution.

After drying over anhydrous sodium sulfate, the solvent was distilled off, leaving 7.2389 grams of crude product.

As a result of gas chromotography by the internal standard method, this crude product was found to contain methyl 3-(4-chlorobenzyl)-1-isopropyl-2-oxocyclopentane carboxylate (I-2) in a yield of 65%.

Reference Preparation Example

Preparation of 2-(4-chlorobenzyl)-5-isopropylcyclopentanone (VII-1)

To a solution of 1.4026 grams (4.27 mmole) of methyl 3-(4-chlorobenzyl)-1-isopropyl-2-oxocyclopentane carboxylate in 1.8 ml of isopropyl alcohol and 0.84 ml of toluene was added 2.36 ml of a 5N sodium hydroxide solution, and the mixture was heated at 80° C for 5 hours.

After cooling, the resulting reaction mixture was admixed with 30 ml of water and then extracted twice with 30 ml of ethyl acetate. The resulting organic layer was washed with water and sodium chloride.

After drying the organic layer over anhydrous sodium sulfate, the solvent was distilled off under reduced pressure, leaving 0.8711 grams of yellow oily material.

As a result of gas chromotography by the internal standard method, this crude product was found to be as pure as 96.34% by weight and to contain 3-(4-chlorobenzyl)-5-isopropylcyclopentanone (VII-1) in a yield of 78.4%.

The following examples are directed to formulations or preparations containing derivative (I) as an active ingredient.

Formulation Example 1 : Dust

| | |
|---|---|
| Compound No. I-1 | 3 parts by weight |
| Clay | 40 parts by weight |
| Talc | 57 parts by weight |

The above-mentioned ingredients were mixed to prepare a dust.

Formulation Example 2 : Wettable Powder

| | |
|---|---|
| Compound No. I-2 | 50 parts by weight |
| Lignin sulfonate | 5 parts by weight |
| Alkyl sulfonate | 3 parts by weight |
| Diatomaceous earth | 42 parts by weight |

The above-mentioned ingredients were mixed to prepare a wettable powder. This preparation was used after diluting it with water.

Formulation Example 3 : Granules

| | |
|---|---|
| Compound I-1 | 5 parts by weight |
| Bentonite | 43 parts by weight |
| Clay | 45 parts by weight |
| Lignin sulfonate | 7 parts by weight |

The above-mentioned ingredients were mixed and kneaded with addition of water. The mixture was granulated by an extrusion granulating machine, followed by drying.

Formulation Example 4 : Emulsion

| | |
|---|---|
| Compound I-2 | 20 parts by weight |
| Polyoxyethylene alkyl aryl ether | 10 parts by weight |
| Polyoxyethylene sorbitan monolaurate | 3 parts by weight |
| Xylene | 67 parts by weight |

Antifungal Tests:

The compounds (I-1) and (I-2) were tested for anti-fungal activities against various plant pathogens.

Test Procedures

Each of the compounds (I-1) and (I-2) was dissolved in dimethylsulfoxide in a suitable concentration, 0.6 ml of the solution was well mixed with 60 ml of a PAS culture medium at about 60° C in a 100 ml conical flask, and the resultant mixture was poured into Petri dishes and was caused to coagulate, by which plate culture media containing compound of this invention were obtained.

Plate culture media on which test fungi were previously cultured were punched by a cork borer so as to have a diameter of 4 mm, followed by inoculating on the above-mentioned plate culture media. After inoculation and incubation for 1 - 3 days at optimum temperature for each fungus, growth of fungi was observed by measuring the diameter of the colony. Hyphae elongation inhibitory rates were determined in accordance with the equation :

$$R = 100\ (dc - dt)/dc$$

where

R = Hyphae elongation inhibitory rate (%)
dc = Diameter of colony on the non-treated plate culture medium
dt = Diameter of colony on the plate culture medium containing the tested compound

Test results were ranked in three stages by the following ranking system.

0: R is lower than 40%;
1: R is between 40% and 80%; and
2: R is higher than 80%.

The results against the test fungi are shown in Table 2.

TABLE 2

| Compound No. | Concentration (μg/ml) | Test Fungi | | |
|---|---|---|---|---|
| | | H.s. | R.s. | S.c. |
| I-1 | 100 | 1 | 1 | 1 |
| I-2 | 100 | 1 | 1 | 1 |

The abbreviations for the test fungi in Table are as follows:

H.s.: Helminthosporium sigmoideum
R.s.: Rhizoctonia solani
S.c.: Sclerotinia sclerotirum

## Claims

1. A 3-(unsubstituted or substituted benzyl)-1-alkyl-2-oxocyclopentane carboxylic acid alkyl ester derivative (I)

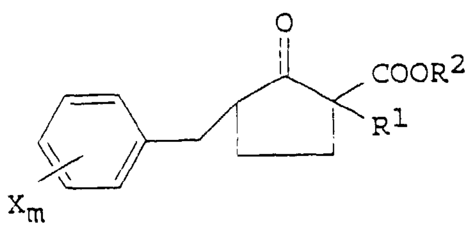

(I)

where $R^1$ and $R^2$ are the same or different $C_1$ - $C_5$ alkyl groups, m is 0 or an integer from 1 to 5, and the or each X, when present, is selected independently from halogen, cyano, $C_1$ - $C_5$ alkyl, $C_1$ - $C_5$ haloalkyl, phenyl and nitro groups.

2. A compound according to claim 1 in which X is Cℓ.

3. A compound according to claim 1 or 2 wherein $R^1$ is methyl or isopropyl, $R^2$ is methyl, X is a chlorine atom, and m is 1.

4. A compound according to claim 1, 2 or 3 wherein X is located at the 4-position of the phenyl group.

5. A method for the preparation of a 3-(unsubstituted or substituted benzyl)-1-alkyl-2-oxocyclopentane carboxylic

acid alkyl ester derivative (I) according to claim 1 which comprises continuous rearrangement and alkylation, in which the rearrangement is carried out to convert derivative (IV):

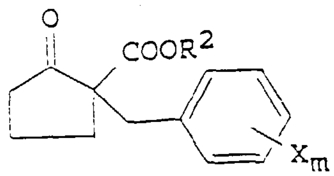

(IV)

into derivative (V)

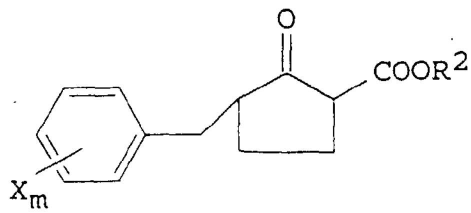

(V)

with alkali metal lower ($C_1$ - $C_5$) alkoxide in lower ($C_1$ - $C_5$) alcohol and the alkylation is carried out after distillation of the lower ($C_1$-$C_5$) alcohol to alkylate resulting compound (V) with alkyl halide (VI)

$$R^1 - Z^1 \qquad (VI)$$

where $R^1$, $R^2$, X and m are as defined in claim 1 and $Z^1$ is a halogen atom.

6. A method according to claim 5 which comprises continuous benzylation followed by said rearrangement and alkylation, in which the benzylation is carried out to benzylate derivative (II)

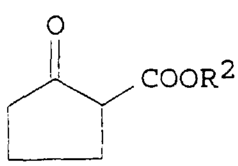

(II)

with unsubstituted or substituted benzyl halide (III)

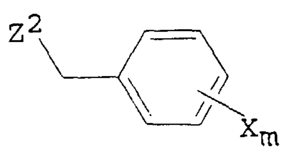

(III)

in the presence of alkali metal base to give the ester (IV), where $Z^2$ is a halogen atom.

7. A method according to claim 5 or 6 further comprising the steps of hydrolysing the resulting derivative (I) and decarboxylating the hydrolysate to form 3-(unsubstituted or substituted benzyl)-1-alkyl-2-oxocyclopentane derivative (VII)

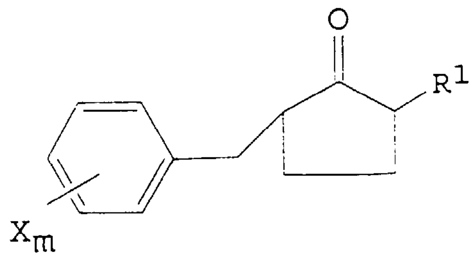

(VII)

8. A fungicide containing as an active ingredient a compound according to any one of claims 1 to 4.

**Patentansprüche**

1. Ein 3-(unsubstituiertes oder substituiertes Benzyl)-1-alkyl-2-oxocyclopentancarbonsäurealkylester-derivat (I):

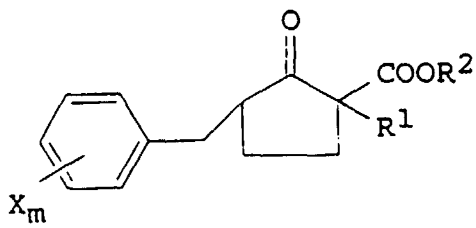

(I)

worin $R^1$ und $R^2$ gleiche oder verschiedene $C_{1-5}$-Alkylgruppen sind, m 0 oder eine ganze Zahl von 1 bis 5 ist und X oder jedes X, sofern vorhanden, unabhängig aus der durch Halogenatome, Cyanogruppen, $C_{1-5}$-Alkylgruppen, $C_{1-5}$-Halogenalkylgruppen, Phenylgruppen und Nitrogruppen gebildeten Gruppe gewählt ist.

2. Verbindung nach Anspruch 1, worin X ein Chloratom ist.

3. Verbindung nach Anspruch 1 oder 2, worin $R^1$ Methyl oder Isopropyl ist, $R^2$ Methyl ist, X ein Chloratom ist und m = 1.

4. Verbindung nach Anspruch 1, 2 oder 3, worin X in 4-Stellung der Phenylgruppe steht.

5. Verfahren zur Herstellung eines 3-(unsubstituierten oder substituierten Benzyl)-1-alkyl-2-oxocyclopentancarbonsäurealkylester-derivats (I) gemäß Anspruch 1, bei dem man kontinuierlich umlagert und alkyliert, wobei man die Umlagerung zur Überführung des Derivats (IV):

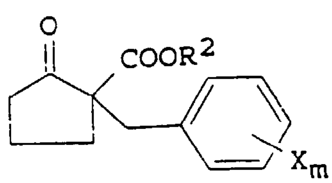

(IV)

in das Derivat (V):

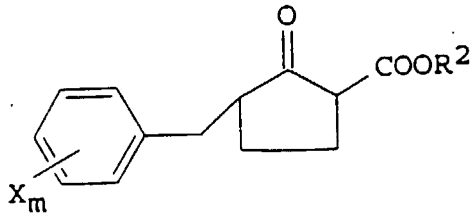

(V)

mit einem Alkalimetall eines niederen $C_{1-5}$-Alkoxids in einem niederen $C_{1-5}$-Alkohol durchführt und die Alkylierung nach Destillation des niederen $C_{1-5}$-Alkohols zur Alkylierung der anfallenden Verbindung (V) mit einem Alkylhalogenid (VI) durchführt:

$$R^1 - Z^1 \qquad \text{(VI)}$$

worin $R^1$, $R^2$, X und m die Bedeutungen gemäß Anspruch 1 besitzen und $Z^1$ ein Halogenatom ist.

**6.** Verfahren nach Anspruch 5, bei dem man kontinuierlich benzyliert und danach umlagert und alkyliert, wobei man die Benzylierung zum Benzylieren von Derivat (II):

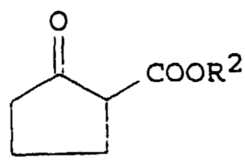

(II)

mit einem unsubstituierten oder substituierten Benzylhalogenid (III):

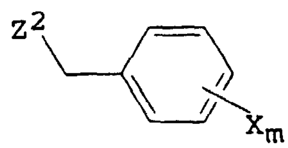

(III)

in Gegenwart einer Alkalimetallbase durchführt und den Ester (IV) erhält, worin $Z^2$ ein Halogenatom ist.

**7.** Verfahren nach Anspruch 5 oder 6, bei dem man ferner stufenweise das resultierende Derivat (I) hydrolysiert und das Hydrolysat decarboxyliert unter Bildung von 3-(unsubstituiertem oder substituiertem Benzyl)-1-alkyl-2-oxo-cyclopentan-derivat (VII):

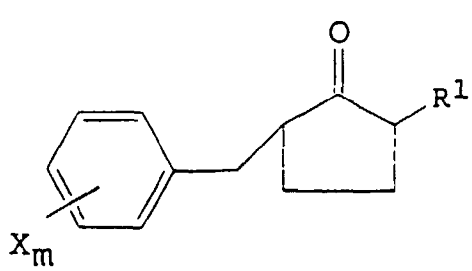

(VII)

**8.** Fungizid mit einem Gehalt an einer Verbindung gemäß einem der Ansprüche 1 bis 4 als aktivem Bestandteil.

## Revendications

1. Dérivé (I) ester alkylique d'acide 3-(benzyle substitué ou non substitué)-1-alkyl-2-oxocyclopentane carboxylique

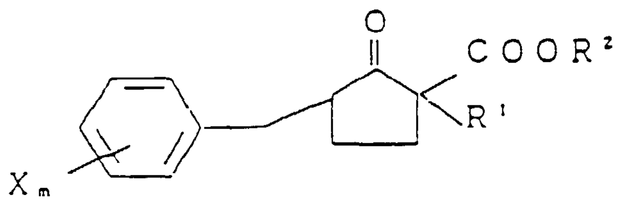

(I)

dans lequel $R^1$ et $R^2$ sont des groupes alkyle en $C_1$ à $C_5$, identiques ou différents, m vaut 0 ou un entier de 1 à 5 et, lorsqu'il est présent, X ou chaque X est choisi indépendamment parmi un halogène, un groupe cyano, un groupe alkyle en $C_1$ à $C_5$, un groupe halogénoalkyle en $C_1$ à $C_5$, un groupe phényle et un groupe nitro.

2. Composé selon la revendication 1, dans lequel X représente Cl.

3. Composé selon la revendication 1 ou 2, dans lequel $R^1$ représente un groupe méthyle ou isopropyle, $R^2$ représente un groupe méthyle, X représente un atome de chlore et m vaut 1.

4. Composé selon la revendication 1, 2 ou 3, dans lequel X est situé en position 4 sur le groupe phényle.

5. Procédé pour la préparation d'un dérivé (I) ester alkylique d'acide 3-(benzyle substitué ou non substitué)-1-alkyl-2-oxocyclopentane carboxylique selon la revendication 1, qui comprend un réagencement et une alkylation en continu, le réagencement étant conduit pour transformer le dérivé (IV):

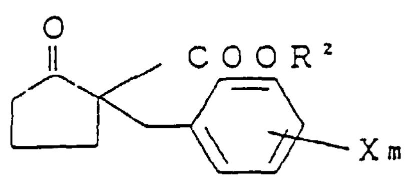

(IV)

en le dérivé (V):

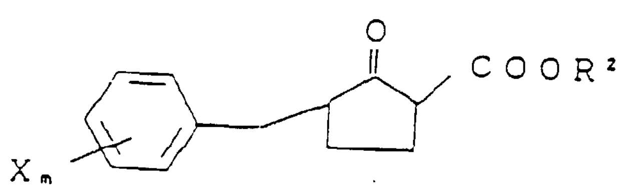

(V)

avec un alcoxy inférieur en $C_1$ à $C_5$, d'un métal alcalin dans un alcool inférieur en $C_1$ à $C_5$, et l'alkylation est conduite après distillation de l'alcool inférieur en $C_1$ à $C_5$, pour alkyler le composé résultant (V) avec un halogénure d'alkyle (VI)

$$R^1\text{-}Z^1 \qquad \text{(VI)}$$

dans lequel $R^1$, $R^2$, X et m sont tels que définis dans la revendication 1, et $Z^1$ est un atome d'halogène.

6. Procédé selon la revendication 5, qui comprend la benzylation en continu, suivie par ledit réagencement et ladite alkylation, dans lequel la benzylation est conduite en vue de benzyler le dérivé (II)

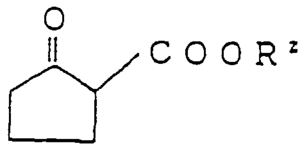

(II)

avec un halogénure de benzyle substitué ou non substitué

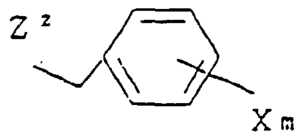

(III)

en présence d'une base de métal alcalin, pour fournir l'ester (IV), $Z^2$ étant un atome d'halogène.

**7.** Procédé selon la revendication 5 ou 6, qui comprend en outre les étapes consistant à hydrolyser le dérivé résultant (I) et à décarboxyler l'hydrolysat pour former le dérivé (VII) de 3-(benzyle substitué ou non substitué)-1-alkyl-2-oxocyclopentane

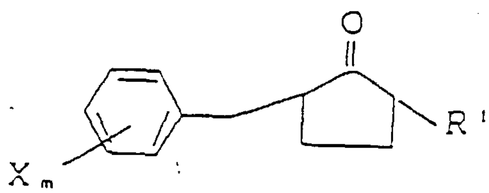

(VII)

**8.** Fongicide contenant comme ingrédient actif un composé selon l'une quelconque des revendications 1 à 4.